# EUROPÄISCHE PATENTSCHRIFT

(11) **EP 2 452 663 B1**
(45) Veröffentlichungstag und Bekanntmachung des Hinweises auf die Patenterteilung: **24.04.2013**
(21) Anmeldenummer: 10015048.1
(22) Anmeldetag: 26.11.2010
(51) Int. Cl.: A61G 12/00, A61G 13/10, F16M 11/04, F16M 11/06, F16M 11/08, F16M 11/20, F16M 13/02

(54) **Trage- bzw. Versorgungssystem für medizintechnische Geräte**
Carrier and supply system for medicinal devices
Système de portage ou d'alimentation pour appareils médicaux

(30) Priorität: 16.11.2010 DE 102010051525
(43) Veröffentlichungstag der Anmeldung: 16.05.2012
(73) Patentinhaber: Ondal Medical Systems GmbH, 36088 Hünfeld (DE)
(72) Erfinder: Volkenand, Kai, 36088 Hünfeld (DE); Ickler, Fritz, 36275 Kirchheim (DE); Perplies, Stefan, 36088 Hünfeld (DE)
(74) Vertreter: Graf von Stosch, Andreas

(56) Entgegenhaltungen:
- EP-A1- 1 785 104
- WO-A1-2009/018422
- US-A1- 2010 223 857
- US-B1- 7 770 860

## Beschreibung

Die vorliegende Erfindung bezieht sich auf eine Konsole zur medizin-technischen Versorgung in einem Behandlungsraum, und insbesondere auf eine Konsole mit mehreren Anschlüssen oder Anschlussdosen zur medizin-technischen Versorgung bzw. zur Entnahme von Gasen und/oder elektrischer Versorgungsströme für medizin-technische Endgeräte.

Die erfindungsgemäße Konsole ist insbesondere zum Montieren bzw. Aufhängen an einem Tragesystem ausgebildet. Daher betrifft die Erfindung sowohl ein medizintechnisches Versorgungssystem als auch ein nachrüstbares und modulares medizinisches Tragesystem.

US 2010/0223857 A1 beschreibt eine zum Montieren an einer vertikale Fläche ausgebildete Konsole.

Tragesysteme und Versorgungskonsolen für medizin-technische Endgeräte sind im klinischen Bereich häufig im Einsatz, insbesondere in Krankenhäusern (wie z.B. in OP-Sälen und an Intensivstationen) und in Arztpraxen. Im OP-Saal arbeiten die Chirurgen und das medizinische Personal unter teilweise sehr schwierigen Bedingungen und ihre Leistung hat bekannterweise unmittelbare Auswirkungen auf die Chancen der jeweiligen Patienten auf eine erfolgreiche Genesung. Zudem ist inzwischen auch bekannt, dass ein Aufenthalt im Krankenhaus, besonders über längere Zeiträume, typischerweise eine negative Auswirkung auf den geistigen Zustand der Patienten hat, was auch nicht als heilungsfördernd gilt, denn Patienten im Krankenhaus fühlen sich oft alleine und isoliert, getrennt von ihrer vertrauten Umgebung und teilweise auch von ihren Familien und Freunden. Die Erfinder haben sich daher mit der Frage beschäftigt, wie solche Versorgungs- und Tragesysteme die medizinischen Ergebnisse und die Heilungschancen der Patienten weiter unterstützen bzw. steigern können.

### Kurze Beschreibung der Erfindung

Diese Aufgabe wird durch eine Konsole gemäß Anspruch 1 gelöst. Bevorzugte Ausführungen der Erfindung ergeben sich aus den Merkmalen der abhängigen Ansprüche.

Die Erfindung sieht eine Konsole zur medizin-technischen Versorgung in einem Behandlungsraum vor, wie z.B. in einem OP-Saal oder an einer Intensivstation, welche Konsole die folgenden Merkmale aufweist:
ein im Behandlungsraum montierbaren Traggestell mit mehreren Anschlüsse bzw. Anschlussdosen zur medizin-technischen Versorgung bzw. zur Entnahme von Gasen und/oder elektrischer Versorgungsströme sowie zur Herstellung elektronischer Kommunikationswege für medizin-technische Endgeräte, und
ein im Traggestell integriertes Audiowiedergabesystem zur Wiedergabe von vorbestimmten wohltuenden bzw. therapeutischen Tönen im Behandlungsraum, bzw. zur Beschallung des Behandlungsraums mit den vorbestimmten Tönen.

Die Erkenntnisse, dass bestimmte Töne, wie z.B. Musik, als wohltuend oder therapeutisch wirken und der Wiederherstellung, Erhaltung und Förderung seelischer, körperlicher und geistiger Gesundheit dienen können, wurden von den Erfindern in der Konzipierung der erfindungsgemäßen Konsole umgesetzt. Bislang hat es an geeigneten Einrichtungen bzw. Gerätschaften für eine sichere, hygienische, schonende, und medizinisch zugelassene Anwendung von solchen Erkenntnissen im stationären und/oder ambulanten klinischen Bereich, insbesondere in Operationssälen und/oder an Intensivstationen gefehlt. Mit der vorliegenden Erfindung wurde ein System geschaffen, das der Gesundheit und der erfolgreichen Genesung der Patienten in zwei verschiedenen Arten und Weisen zugute kommt. Erstens kann das integrierte Audiosystem eine oder mehrere, für den operierenden Chirurg und/oder für sein Team von medizinischem Personal vorbestimmten Aufnahmen abspielen, die dann besonders beruhigend oder konzentrationsfördernd auf den Chirurg bzw. das Personal auswirken können, um somit die ärztliche bzw. medizinische Leistung zu steigern. Zweitens können vorbestimmte Töne bzw. Musik im stationären klinischen Bereich, z.B. an der Intensivstation, auch positiv auf die seelische, körperliche und geistige Gesundheit der Patienten auswirken und dabei der negativen geistigen Effekte eines Aufenthalts im Krankenhaus entgegenwirken. Selbst wenn ein Patient in einem Koma liegt, können die vorbestimmten Töne trotzdem noch subliminal oder unterbewusst auf den Patienten positiv auswirken.

Die vorbestimmten Töne werden vorzugsweise an den Patienten bzw. an das medizinische Personal genau angepasst bzw. für sie ausgewählt, um eine maximale positive Wirkung hervorzurufen. Hierbei können die Töne eine bestimmte Wunschmusik oder andere vorbestimmte Aufnahmen sein, wie z.B. Meer- oder Naturgeräusche. Mit der erfindungsgemäßen Konsole kann sich einerseits die beruhigende, schmerzlindernde, entspannende und heilungsfördernde Wirkung von Tönen auf den Patienten und anderseits die beruhigende Wirkung von Musik auf den Chirurgen nutzbar machen, um dabei die medizinischen Ergebnisse und die Heilungschancen zu unterstützen und ggf. zu steigern.

Das Audiowiedergabesystem der erfindungsgemäßen Konsole ist ein medizinisch zugelassenes Gerät und weist mehrere, in verschiedenen Richtungen orientierte Lautsprecher auf. Die Beschallung des Behandlungsraums ist bevorzugt als Hintergrundsbeschallung konzipiert. Allerdings können die Lautsprecher in Kopfhörern integriert werden, sodass die wohltuenden bzw. therapeutischen Töne nur für bestimmte Zuhörer (also, diejenigen, die die Kopfhörer tragen) im Behandlungsraum zugänglich sind.

In einer bevorzugten Ausführungsform der Erfindung umfasst das Traggestell mehrere Module. Die Module sind vorzugsweise genormte Bauteile, die eine Grundform teilen und miteinander im Traggestell austauschbar sind. Die Anschlüsse bzw. Anschlussdosen sind in mindestens einem, bevorzugt in mehreren der Module vorgesehen. Ferner befindet sich das Audiosystem in mindestens einem, und bevorzugt in mehreren der Module der Versorgungskonsole. Zum Beispiel weist das Audiosystem mehrere Lautsprecher auf, die sich vorzugsweise in mehreren Modulen des Traggestells befinden. Die mehreren Modulen des Traggestells sind bevorzugt nebeneinander in einer oder mehreren Reihen zusammengebaut. Vorzugsweise weist mindestens eines der Module ein Gehäuse auf, das Teile des Audiowiedergabesystems aufnimmt und zumindest teilweise, bevorzugt im Wesentlichen vollständig, schützend umschließt bzw. ummantelt. Die Module führen zu einem sehr flexiblen Aufbau der Konsole und ermöglichen eine kostengünstige Vorfertigung bzw. Konfektion sowie eine einfache Montage und einfache Wartungsarbeiten. Hierzu ist das Audiosystem derart in den Modulen untergebracht, dass die äußeren Oberflächen des Audiosystems, wie z.B. der Lautsprechermodulen, sehr leicht zu reinigen bzw. desinfizieren. Mit anderen Worten lassen sich die äußeren Oberflächen des Audiosystems in der erfindungsgemäßen Konsole mit Desinfizierungsmittel reinigen, ohne das Audiosystem zu beeinträchtigen.

In einer bevorzugten Ausführungsform der Erfindung weist das Traggestell einen Rahmen bzw. einen Kern auf, der zur Montage bzw. zur Aufhängung an einem Deckenstativ ausgebildet ist. Die mehreren Module sind vorzugsweise seitlich an den Rahmen bzw. Kern angebracht und befestigt. Vorzugsweise hat das Traggestell eine längliche Form und ist im Wesentlichen senkrecht angeordnet, so dass die mehreren Modulen übereinander angeordnet sind. In einer besonders bevorzugten Ausführungsbeispiel sind an einer Seite des Traggestells eine erste im wesentlichen senkrecht angeordnete Säule und an einer zweiten gegenüberliegenden Seite des Traggestells eine zweite im wesentlichen senkrecht angeordnete Säule bestehend aus jeweils übereinander angeordneten Modulen angeordnet.

In einer bevorzugten Ausführungsform der Erfindung verfügt das Audiowiedergabesystem über mindestens eine Stelle zur Aufnahme eines Ton- bzw. Musikspeichermediums, wie z.B. Diskette, CD, Speicherstab (i.e. "Memory Stick") oder ähnliche. Die mindestens eine Stelle zur Aufnahme des Ton- bzw. Musikspeichermediums gestaltet sich daher nach den herkömmlichen Speichermedien. Vorzugsweise weist das Audiowiedergabesystem eine Steuereinheit oder eine Aufnahmestelle zum Aufnehmen bzw. zum Andocken einer Steuereinheit auf, mit welcher Steuereinheit der Benutzer das Abspielen der Töne oder Musik steuern bzw. bedienen kann. In diesem Zusammenhang kann die Steuereinheit ein digitales Abspiel- und Speichergerät sein, wie z.B. ein "iPod", "iPhone" oder ein mp3-Gerät, und die erfindungsgemäße Konsole kann eine Aufnahmestelle bzw. Schnittstelle dafür beinhalten, die entsprechend ausgebildet ist - z.B. als Klinkensteckereingang, so dass die Steuereinheit beliebig eingesetzt bzw. entfernt werden kann. Alternativ kann die Steuereinheit in einer der Module integriert bzw. fixiert sein. Wunschgemäß kann die Konsole auch mit einem Radio-Tuner ausgestattet sein.

In einer bevorzugten Ausführungsform der Erfindung ist das Audiosystem mittels der Steuereinheit programmierbar, um die Töne oder die Musik auszuwählen bzw. vorzubestimmen. Vorzugsweise umfasst die Steuereinheit des Audiowiedergabesystems einen Audioverstärker bzw. einen Beschallungsverstärker. Ferner weist die Steuereinheit vorzugsweise eine Fernbedienung auf, mit der man mindestens die vorbestimmten wohltuenden bzw. therapeutischen Tönen, die Programmierung und/oder die Lautstärke ändern sowie das Audiosystem ein- und ausschalten kann.

In einer bevorzugten Ausführungsform der Erfindung hat das Audiosystem mittels der Steuereinheit einen Internet-Anschluss, so dass man die vorbestimmten Tönen bzw. die Wunschmusik aus dem Internet direkt herunterladen kann. Hierfür kann die Steuereinheit bspw. einen Prozessor und die nötige Software, wie z.B. Webbrowser-Software, beinhalten. Der Internet-Anschluss kann kabellos erfolgen, z.B. über eine WLAN-Verbindung.

Gemäß eine weitere Ausführungsform sieht die Erfindung ein Tragesystem für medizinische Geräte vor, wobei das Tragesystem zur Montage bzw. zur Aufhängung an einem Deckenstativ in einem Behandlungsraum ausgebildet ist und ein Traggestell mit einem integrierten medizinisch zugelassenen Audiowiedergabesystem zur Beschallung des Raums umfasst. Das Traggestell Teil ist einer Konsole gemäß Ansprüch 1 bis 9, die ggf. in Form einer Versorgungsbrücke sein kann, welche fest oder beweglich mit der Decke verbunden ist. Vorzugsweise umfasst das Tragesystem ein Stativ zum Aufhängen bzw. Montieren des Systems an einer Decke im Behandlungsraum.

Das Aufstellen oder die sichere und hygienische Positionierung einer herkömmlichen Audioanlage in einen OP-Saal oder an eine Intensivstation war bisher nahezu unmöglich. Ferner hätte eine solche Aktion zu Patientenschäden führen können und die Verwendung von solchen herkömmlichen Geräten erscheint aufgrund fehlender medizinischer Zulassung sowieso höchst fraglich bzw. unzulässig gewesen zu sein. Die erfindungsgemäße Integration des Audiosystems in der Konsole bzw. im Tragesystem hingegen ermöglicht eine medizinische Zulassung und eine sichere und hygienische Positionierung in allen Bereichen des Krankenhauses, so dass die Erkenntnisse der wohltuenden bzw. therapeutischen Tönen im OP-Saal und im stationären oder ambulanten klinischen Bereich umgesetzt werden können.

### Kurze Beschreibung der Zeichnungen

Weitere bevorzugte Ausgestaltungen der Erfindung ergeben sich aus der nachfolgenden Beschreibung von Ausführungsbeispielen, die unter Bezugnahme auf die beigefügten Figuren erfolgt, wobei einander funktionsgleiche oder funktionsähnliche Bauteile mit denselben Bezugszeichen gekennzeichnet sind. Es zeigt:
- Figur 1: perspektivische Ansicht einer Versorgungskonsole gemäß einem Ausführungsbeispiel der Erfindung;
- Figur 2: perspektivische Ansicht eines Moduls der erfindungsgemäßen Konsole in Figur 1;
- Figur 3: perspektivische Ansicht eines weiteren Moduls der erfindungsgemäßen Konsole in Figur 1; und
- Figur 4: perspektivische Ansicht eines Tragesystems gemäß einem Ausführungsbeispiel der Erfindung.

### Beschreibung der Ausführungsbeispiele der Erfindung

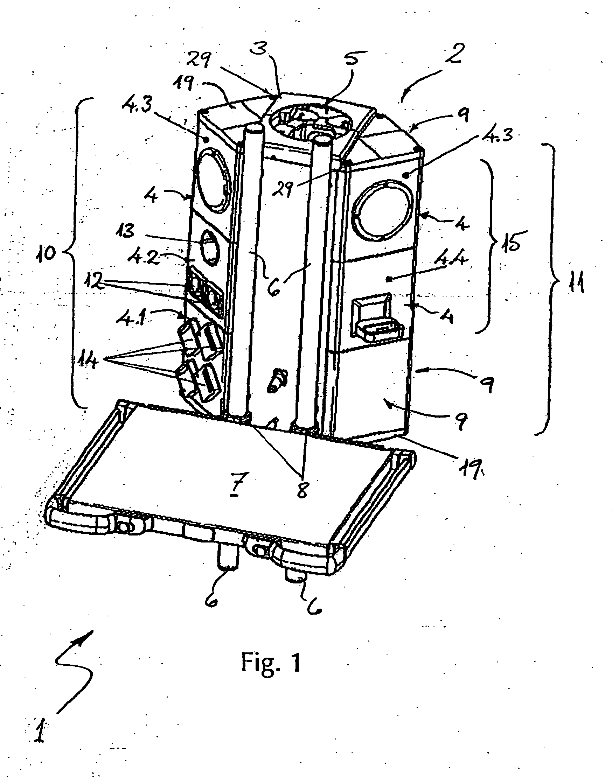

Die Figur 1 zeigt eine erfindungsgemäße Konsole 1 zur Versorgung von medizin-technischen bzw. medizin-elektrischen (ME) Endgeräte (nicht gezeigt) mit Gas, Strom und/oder Kommunikationstechnik. Die Versorgungskonsole 1 weist ein Traggestell 2 auf, das mittels eines Deckenstativs zum Montieren bzw. Aufhängen an der Decke in einem Behandlungsraum (z.B. in einem OP-Saal oder ICU-Raum (Intensivstation)) in einem Krankenhaus ausgebildet ist. Hierzu umfasst das Traggestell 2 einen Rahmen oder Kern 3 und seitlich angeordnete Module 4, die u.a. Anschlussdosen bzw. Steckeranschlüsse zur medizin-technischen Versorgung bzw. zur Entnahme von Gasen und/oder elektrischer Versorgungsströme sowie zur Herstellung elektronischer Kommunikationswege für die ME-Endgeräte beinhalten. Der Rahmen oder Kern 3 des Traggestells 2 ist zentral angeordnet, erstreckt sich im Wesentlichen vertikal zwischen den seitlichen Modulen 4 und hat an seiner oberen Seite eine zentrale Öffnung 5, die als Montagepunkt zur Befestigung mit einem Deckenstativ dient. Zudem weist das Traggestell 2 zwei sich vertikal erstreckende Rohre 6 auf, die mit dem zentralen Rahmen bzw. Kern 3 des Gestells 2 fest verbunden sind und an denen ein Tablett 7 zum Aufnehmen der ME-Geräte mittels zwei Klemmbacken 8 höhenverstellbar befestigt ist.

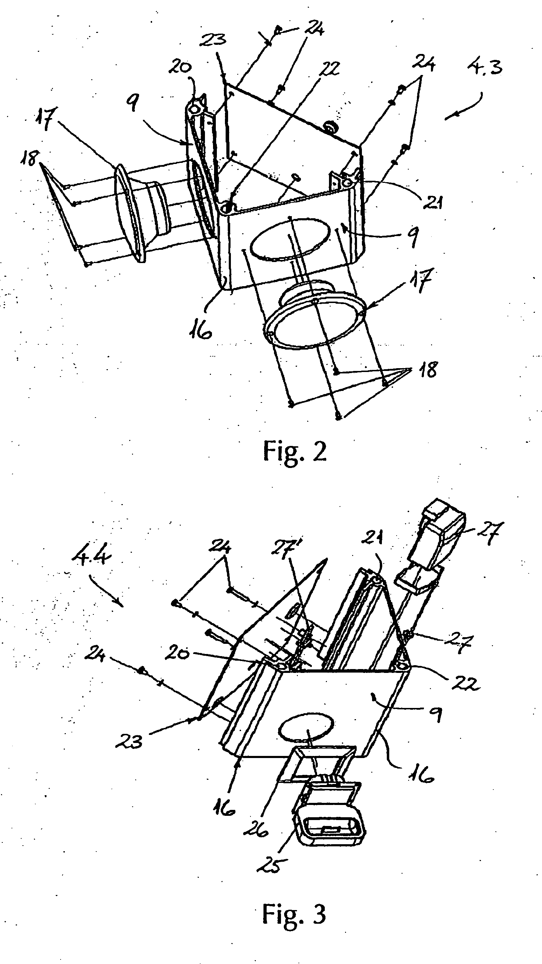

In ihrer Grundform sind die Module 4 genormte Bauteile, die miteinander auswechselbar sind. Jedes der seitlich angeordneten Module 4 in diesem Ausführungsbeispiel hat eine dreieckige Form und präsentiert zwei angewinkelte Seitenflächen 9, wie später mit Bezug auf die Figuren 2 und 3 näher beschrieben wird. Die Module 4 sind derart an den Seiten des Traggestells 2 angeordnet, dass sie an einer ersten Seite des Traggestells 2 eine erste im wesentlichen senkrecht angeordnete Säule 10 und an einer zweiten gegenüberliegenden Seite des Traggestells 2 eine zweite im wesentlichen senkrecht angeordnete Säule 11 aus jeweils übereinander angeordneten Modulen 4 bilden.

Obwohl jedes Modul mit derselben Grundform versehen ist, ermöglichen die Modulen 4 unterschiedliche Ländervarianten von Stromsteckdosen (erste Anschlussdosen 12 - alle international gängigen Steckdosentypen sind einbaubar), unterschiedliche Bauformen von Gasentnahmedosen (zweite Anschlussdosen 13) und unterschiedliche Bauformen von Kommunikationselektronikanschlüssen (dritte Anschlussdosen 14 - z.B. für eine Videokamera, Monitor usw.) aufzunehmen. Durch das wahlweise Zusammenfügen einzelner Modulen 4.1, 4.2 wird eine Vielfalt von Konfigurationsmöglichkeiten erzielt. Unter Berücksichtigung von aktuellen Sicherheitsbestimmungen lassen sich wahlweise erste, zweite und dritte Anschlussdosen 12, 13, 14 in einer ersten bzw. zweiten Säule 10, 11 vormontiert (auch komplett mit der Konsole 1 und wahlweise auch mit einem Deckenstativ 30) dem Anwender bereitstellen. Die Module 4.1, 4.2 bestehen aus einem dreieckförmigen Abschnitt eines Strangprofils, welches die Seitenwände 9 gleicher Breite in einem rechten Winkel aufweist. In dem Abschnitt bzw. in den Seitenwänden 9 sind die Anschlussdosen 12 für Stromversorgung und/oder Anschlussdosen 13 für eine Gasentnahme und/oder Anschlussdosen 14 für elektronische Kommunikationstechnik je nach Wunsch und Anwendung eingebaut.

Erfindungsgemäß weist die Konsole 1 ein im Traggestell 2 integriertes Audiowiedergabesystem 15 zur Beschallung des Behandlungsraums mit vorbestimmten wohltuenden bzw. therapeutischen Tönen, bspw. in Form ausgewählter bzw. vorprogrammierter Musik. In diesem Ausführungsbeispiel ist das Audiosystem 15 in verschiedenen Modulen 4.3, 4.4 des Traggestells 2 untergebracht. Mit anderen Worten setzt sich das integrierte Soundsystem, wie bereits in Figur 1 dargestellt, aus mehreren einzelnen Modulen 4.3, 4.4 zusammen, welche ggf. auch als kombiniertes Modul ausführbar sind.

Eine Darstellung des Moduls 4.3 zur Wiedergabe von akustischen Signalen, hier als Lautsprecher-Modul, ist in Figur 2 dargestellt. Basis des Moduls 4.3 stellt ein dreieckförmiger Grundkörper 16 dar, welcher zur Aufnahme von einem oder mehreren (in diesem Fall zwei) Lautsprechern 17 ausgelegt ist. Die Befestigung von jedem Lautsprecher 17 kann mittels kraft-, stoff- oder formschlüssigen Elementen (z.B. Befestigungsschrauben 18), ähnlichen Befestigungsarten oder einer Kombination derer erfolgen. Der Grundkörper 16 besteht aus einem Abschnitt eines Strangprofils, das eine erste und eine zweite Seitenwand 9 gleicher Breite in einem rechten Winkel aufweist. Wenn die zwei Lautsprecher 17 in den angewinkelten Seitenwänden 9 aufgenommen sind, haben sie damit unterschiedliche Ausrichtungen. In einem Endbereich der einen Seitenwand 9 ist ein erster Kanal 20 und in einem Endbereich der anderen Seitenwand 9 ist ein zweiter Kanal 21 und in einem Kanten-Bereich einer Winkelkante ist ein dritter Kanal 22 jeweils mit einem kreisförmigen Querschnitt angeordnet. Diese Kanäle 20-22 dienen zur Aufnahme jeweils einer Gewindestange zum Verbinden der Modulen über eine obere und untere Abdeckplatte 19 (s. Fig. 1). Das Volumen des Grundkörpers 16 wird mittels einer Abdeckung bzw. Blech 23 geschlossen und die Befestigung an den Grundkörper 16 kann mittels kraft-, stoff-oder formschlüssigen Elementen (z.B. Befestigungsschrauben 24), ähnlichen Befestigungsarten oder einer Kombination derer erfolgen. Alternativ können das Strangprofil und die Abdeckung kombiniert als Grundkörper ausgeführt sein. Die elektrische Verbindung der verbauten Komponenten erfolgt mittels definierten Kabeln, welche mittels vorgegebener Verdrahtung eine Mono-, Stereo- oder "Surround"-Wiedergabe gewährleisten. Zusätzlich ist der Anschluss eines separat aufgehängten Lautsprechers (z.B. in Form eines Kugellautsprechers) im Behandlungsraum möglich.

In Figur 3 ist ein Modul 4.4 zur Steuerung bzw. Verstärkung des Audiowiedergabesystems 15 dargestellt, welches Modul 4.4 als eine Docking-/Verstärkerstation zur Wiedergabe der Wunschmusik dient. Das gesamte Modul 4.4 setzt sich wieder aus einem Grundkörper 16 und einer Aufnahmestelle bzw. einer Dockingstation 25 mit Schnittstelle zum Andocken einer Steuereinheit oder Sonderausführung derer zusammen. Die Aufnahmestelle bzw. Dockingstation 25 kann mittels kraft-, stoff- oder formschlüssigen Elementen (z.B. Steckdosenrahmen 26 in Verbindung mit Leerdosen), ähnlichen Befestigungsarten oder einer Kombination derer erfolgen. Zur elektrischen Spannungsversorgung ist ein Netzteil 27 innerhalb des Moduls 4.4 verbaut, welches intern über die Versorgungskonsole 1 oder über eine externe Spannungsversorgung gespeist wird. Die Befestigung vom Netzteil 27 kann beispielsweise am Grundkörper 16 mittels eines zusätzlichen Befestigungselements oder kraft-, stoff-oder formschlüssigen Elementen erfolgen. Die interne Verdrahtung 27' wird mittels definierten Kabeln zur elektrischen Signalübertragung sichergestellt. Dabei ist ein Mono- oder Stereo- oder "Surround"-Betrieb möglich. Als Schnittstelle zur Verbindung mit dem dargestellten Element in der Figur 3 wird durch elektrische Schnittstellen sichergestellt und ermöglicht. Die Ansteuerung von der Steuereinheit ist direkt am Element, mittels Fernbedienung F oder über andere Möglichkeiten (z.B. ein "Touchscreen") gegeben und zulässig.

Trennplatten (nicht gezeigt) zwischen den übereinander angeordneten Modulen 4 können als Einlegeteil dazu dienen, die abgesägten Strangprofile untereinander zu zentrieren. Des Weiteren werden Toleranzen, die sich beim Absägen einstellen, dadurch kaschiert. Diese Trennplatten können ebenfalls dazu dienen, eine gasdichte Trennung zwischen zwei Moduln 4.1 - 4.4 sicherzustellen, die untereinander durch die Trennplatten und die Abdeckplatten 23 separiert sind.

Mit Hilfe unterschiedlich bestückter Modulen 4.1 - 4.4 lassen sich unterschiedliche Bestückungskonfigurationen von Säulen 11, 12 baukastenartig realisieren, wobei ein System von Modulen unterschiedlich hoher Abschnitte vorgesehen werden können. Die Anzahl der Module 4 ist frei wählbar und unabhängig vom Tragsystem für Tabletts 7 und vom Geräteträger. Gas- und elektrische Anschlüsse sowie Lautsprecher-und Steuereinheiten des Audiosystems 15 können ggf. auf einer Seite (z.B. nur nach vorn bzw. nach hinten) montiert werden. Die Dreiecksform der Modulen 4.1 - 4.4 ergeben Vorteile hinsichtlich Ergonomie, visueller Kontrolle und Kabel-/Schlauchverlegung bzw. Anschlussbelegung sowie hinsichtlich der Bedienung des Audiosystems 15 und der Beschallung des OP- oder ICU-Raumes. Außerdem ist keine Festlegung bezüglich des Einbauorts vorn oder hinten bzw. linke oder rechte Seite notwendig und die Stromanschlüsse 12, Gasanschlüsse 13 und Kommunikationsanschlüsse 14 sind wahlweise kombinierbar einbaubar.

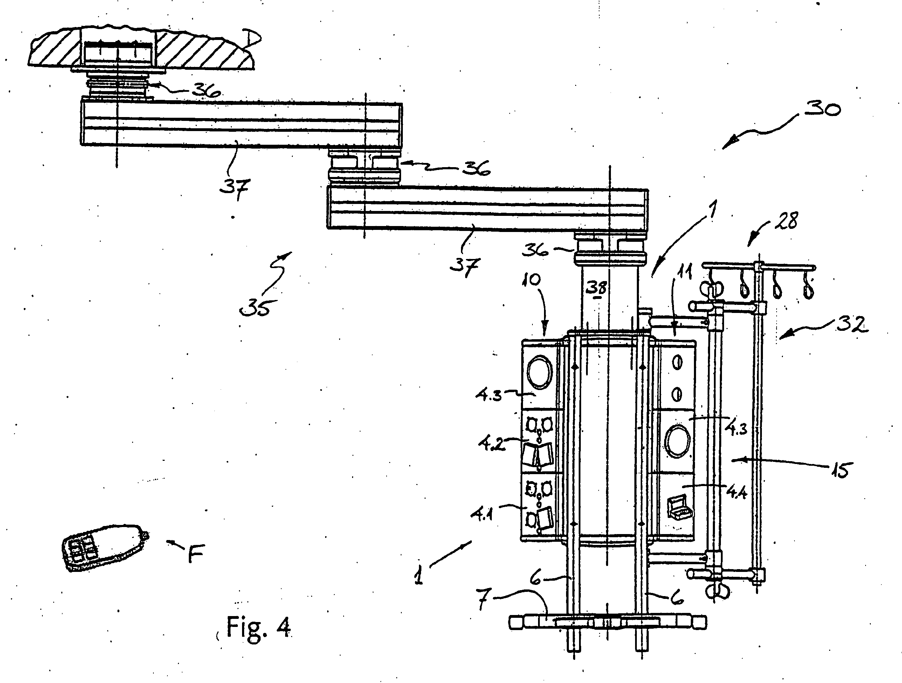

Eine oder auch zwei Säulen 10, 11 montiert an dem Traggestell 2, ergeben eine medizin-technische Versorgungseinheit (Konsole 1), die dem Anwender ermöglicht, medizinische Gase, elektrische Stromversorgung, Kommunikationstechnik sowie Audiowiedergabe zur Verfügung zu haben. Die Gestaltung bzw. Ausführung wird vom Anwender festgelegt. Die einzelnen Moduln 4.1 - 4.4 werden mit den entsprechenden Anschlussdosen 12, 13, 14 bestückt und verschraubt. Entsprechende Leitungen werden angeschlossen und gemäß Qualitätsvorgaben geprüft. Unterschiedlich bestückte Modulen 4.1 - 4.4 werden mit Hilfe von Trennplatten untereinander ausgerichtet und montiert. Mehrere Moduln 4.1 - 4.4 sind durch eine Säule 10, 11 zusammengefasst, die oben und unten mit einer Abdeckplatte 19 verschlossen werden. Gewindestangen inkl. Verschraubungen verbinden die Säulen 10 ,11 form- und kraftschlüssig miteinander. Diese montierte Einheit wird an dem Rahmen oder Kern 3 des Traggestells 2 montiert, um anschließend, vervollständigt mit Tablett 7 und ggf. Infusionsstange 28 (s. Figur 4), als kundenspezifische medizintechnische Versorgungskonsole 1 ausgeliefert zu werden. Für Service bzw. Inspektionen können die Säulen 10, 11 mittels einer Drehlager 29 aufgeschwenkt werden.

Die Figur 4 zeigt in einer Seitenansicht ein Tragesystem 30, bei dem die komplette Konsole 1 an einem Deckenstativ 35 mit mindestens einem Tablett 7 und mit einer Halterung 32 für eine Infusionsstange 28. Die Konsole 1 ist zur Positionierung in drei-dimensionalem Raum (in X-Y-Z-Richtungen) mit dem Deckenstativ 35 über den Montagepunkt 5 verbunden. Wie der Figur 4 zu entnehmen ist, kann die Versorgungskonsole 1 an Deckenstative (wie z.B. ein Auslegersystem, oder ein Auslegersystem kombiniert mit höhenverstellbaren Stativen) montiert bzw. als separate Säule ausgeführt sein. Das Deckenstativ bzw. Auslegersystem 35 in diesem Ausführungsbeispiel weist drei Drehlager 36, zwei schwenkbare Arme 37 und eine höhenverstellbare Säule 38 auf. Ebenfalls ist die Integration in einer Versorgungsbrücke, welche fest oder beweglich mit der Decke D verbunden wird, möglich. Als weitere Variante ist die Ausführung des Audiosystems 15 als separate Einheit möglich, welche man an eine andere Position des Ausleger- bzw. Tragarmsystems 35 parallel zur Versorgungskonsole bzw. getrennt im OP- oder ICU-Raum montieren kann.

Mit der vorliegenden Erfindung wird ein medizinisch zugelassenes Audiosystem 15 zur Installation in einer medizinischen Versorgungskonsole 1 (welche im OP- oder Intensivbereich montiert ist) zum Abspielen von Wunschmusik (oder optional von Radioprogrammen mittels Tuner) zur Hintergrundbeschallung während einer Operation bzw. auf der Intensivstation mittels eines in der Versorgungskonsole integrierten, in vier Richtungen abstrahlenden Lautsprecher-Systems und/oder eines extern montierten Kugellautsprechersystem (in 4-Richtungen abstrahlend mit "Subwoofer") oder eines angeschlossenen Kopfhörer bereit gestellt. Das Audiosystem 15 weist eine in der Konsole installierten Dockingstation 25 auf, welche zur Aufnahme eines iPods oder iPhones über eine genormte Schnittstelle bzw. einen sonstigen Klinkensteckeranschluss geeignet ist, welche als bevorzugtes Merkmal auch über eine Ladeeinrichtung für das iPod oder das iPhone verfügen kann.

Die Module 4.3 - 4.4 des Audiosystems 15 bestehen aus speziellem antibakteriellen und reinigungsmittelbeständigen Material. Sie können mittels Infrarotfernbedienung F oder manuell bedient werden und verfügen über die medizinische Zulassung, sodass sie sicher, hygienisch und zuverlässig in der Patientenumgebung eingesetzt werden dürfen.

## Patentansprüche

1. Konsole (1) zur Versorgung von medizin-technischen Geräten in einem Behandlungsraum, aufweisend
ein zum Montieren im Behandlungsraum ausgebildetes Traggestell (2) mit mehreren Anschlüsse oder Anschlussdosen (12, 13, 14) zur medizin-technischen Versorgung und
ein im Traggestell (2) integriertes Audiowiedergabesystem (15) zur Beschallung des Behandlungsraums mit vorbestimmten wohltuenden bzw. therapeutischen Tönen,
**dadurch gekennzeichnet, dass** das Audiowiedergabesystem (15) mehrere, in verschiedenen Richtungen orientierte Lautsprecher (17) aufweist.

2. Konsole (1) nach Anspruch 1, wobei das Traggestell (2) mehrere Module (4) umfasst, wobei die Anschlüsse bzw. Anschlussdosen (12, 13, 14) in mindestens einem, bevorzugt in mehreren der Module (4) vorgesehen sind, und wobei sich das Audiowiedergabesystem (15) in mindestens einem, und bevorzugt in mehreren der Module (4.3, 4.4) befindet.

3. Konsole (1) nach Anspruch 2, wobei sich die Lautsprecher (17) in mehreren Modulen (4.3) des Traggestells (2) befinden.

4. Konsole (1) nach Anspruch 2 oder Anspruch 3, wobei die mehreren Modulen (4) des Traggestells (2) nebeneinander in einer oder mehreren Reihen angeordnet bzw. zusammengebaut sind.

5. Konsole (1) nach einem der vorhergehenden Ansprüche, wobei das Audiowiedergabesystem (15) mindestens eine Steuereinheit oder eine Stelle (25) zum Andocken einer Steuereinheit aufweist, wobei die Steuereinheit vorzugsweise ein digitales Speicher- und/oder Spielermedium umfasst, wie z.B. ein "iPod", "iPhone" oder ein mp3-Gerät.

6. Konsole (1) nach einem der vorhergehenden Ansprüche, wobei das Traggestell (2) einen Rahmen bzw. einen Kern (3) aufweist, der zur Montage bzw. zur Aufhängung an einem Deckenstativ ausgebildet ist, wobei die mehreren Module (4) seitlich an den Rahmen bzw. Kern (3) angebracht bzw. befestigt sind.

7. Konsole (1) nach einem der vorhergehenden Ansprüche, wobei das Traggestell (2) eine längliche Form aufweist und im Wesentlichen senkrecht angeordnet ist, wobei die mehreren Modulen (4) übereinander angeordnet sind.

8. Konsole (1) nach einem der vorhergehenden Ansprüche, wobei mindestens eines der Module ein Grundkörper oder Gehäuse (16) aufweist, das Teile des Audiowiedergabesystems (15) aufnimmt und zumindest teilweise umgibt.

9. Konsole (1) nach einem der vorhergehenden Ansprüche, wobei an einer Seite des Traggestells (2) eine erste im wesentlichen senkrecht angeordnete Säule (10) und an einer gegenüberliegenden Seite des Traggestells (2) eine zweite im wesentlichen senkrecht angeordnete Säule (11) bestehend aus jeweils übereinander angeordneten Modulen (4) angeordnet sind.

10. Tragesystem (30) für medizinische Geräte, wobei das Tragesystem zur Montage bzw. zur Aufhängung an einem Deckenstativ (35) in einem Behandlungsraum ausgebildet ist und eine Konsole (1) nach einem der vorhergehenden Ansprüche umfasst.

11. Tragesystem (30) nach Anspruch 10, mit einem Stativ (35) zum Aufhängen bzw. Montieren des Systems an einer Decke (D).

12. Tragesystem (30) nach Anspruch 10 oder 11, wobei das Audiosystem (15) zur Hintergrundbeschallung des Behandlungsraums ausgebildet ist, und/oder wobei das Audiosystem (15) zur Wiedergabe bzw. Ausstrahlung der wohltuenden bzw. therapeutischen Töne über in Kopfhörern integrierte Lautsprecher ausgebildet ist, sodass die wohltuenden bzw. therapeutischen Töne nur für bestimmte Zuhörer im Behandlungsraum zugänglich sind.

## Claims

1. Console (1) for supplying medical devices in a treatment room, comprising:
- a support frame (2) adapted to be mounted in the treatment room and having several connectors or sockets (12, 13, 14) for medical support, and
- an audio playback system (15) integrated in the support frame (2) for filling the treatment room with predetermined soothing or therapeutic sounds,
**characterized in that** the audio playback system (15) comprises several loudspeakers (17) oriented in different directions.

2. Console (1) according to claim 1, wherein the support frame (2) comprises several modules (4), wherein the connectors or sockets (12, 13, 14) are provided in at least one module (4), preferably in several modules (4), and wherein the audio playback system (15) is located in at least one of the modules (4.3, 4.4), preferably in several of the modules (4.3, 4.4).

3. Console (1) according to claim 2, wherein the loudspeakers (17) are located in several modules (4.3) of the support frame (2).

4. Console (1) according to claim 2 or claim 3, wherein the several modules (4) of the support frame (2) are arranged or assembled next to each other in one or several rows.

5. Console (1) according to any one of the preceding claims, wherein the audio playback system (15) comprises at least one control unit or a spot for docking a control unit, wherein the control unit preferably comprises a digital memory and/or player media, such as an "iPod", "iPhone" or an mp3 device.

6. Console (1) according to any one of the preceding claims, wherein the support frame (2) comprises a frame or core (3) which is adapted for being mounted to or suspended from a ceiling mount, wherein the several modules (4) are laterally attached or fixed to the frame or core (3).

7. Console (1) according to any one of the preceding claims, wherein the support frame (2) exhibits a longitudinal shape and is arranged substantially vertically, wherein the several modules (4) are arranged one above another.

8. Console (1) according to any one of the preceding claims, wherein at least one of the modules comprises a base body or casing (16) which houses and at least partially surrounds components of the audio playback system (15).

9. Console (1) according to any one of the preceding claims, wherein a first column (10) being substantially vertically arranged is arranged at one side of the support frame (2), and wherein a second column (11) being substantially vertically arranged is arranged at an opposite side of the support frame (2), both columns (10, 11) being comprised of modules (4) that are arranged one above another.

10. Support system (30) for medical devices, wherein the support system is adapted for being mounted to or suspended from a ceiling mount (35) in a treatment room and comprises a console (1) according to any one of the preceding claims.

11. Support system (30) according to claim 10 having a mount (35) for suspending the system from or mounting the system to a ceiling (D).

12. Support system (30) according to claim 10 or 11, wherein the audio system (15) is adapted for filling the treatment room with background sound, and/or wherein the audio system (15) is adapted for playing back or emitting the soothing or therapeutic sounds via loudspeakers integrated in headphones, so that the soothing or therapeutic sounds are available for certain listeners only in the treatment room.

## Revendications

1. Console (1) dévolue à l'alimentation d'appareils médico-techniques dans une salle de traitement, comportant
un bâti de support (2) réalisé en vue du montage dans ladite salle de traitement, et muni de plusieurs raccordements ou boîtes de raccordement (12, 13, 14) dédié(e)s à l'alimentation médico-technique, et
un système (15) de restitution audio, intégré dans ledit bâti de support (2) en vue de la sonorisation de ladite salle de traitement par des sonorités prédéterminées, respectivement bénéfiques ou thérapeutiques,
**caractérisée par le fait que** le système (15) de restitution audio comprend plusieurs haut-parleurs (17) orientés dans des directions différentes.

2. Console (1) selon la revendication 1, dans laquelle le bâti de support (2) comprend plusieurs modules (4), les raccordements ou boîtes de raccordement (12, 13, 14) étant prévu(e)s dans au moins l'un, de préférence dans plusieurs desdits modules (4), et le système (15) de restitution audio étant situé dans au moins l'un et, de préférence, dans plusieurs desdits modules (4.3, 4.4).

3. Console (1) selon la revendication 2, dans laquelle les haut-parleurs (17) sont situés dans plusieurs modules (4.3) du bâti de support (2).

4. Console (1) selon la revendication 2 ou la revendication 3, dans laquelle les multiples modules (4) du bâti de support (2) sont respectivement disposés ou regroupés les uns à côté des autres, en une ou plusieurs rangée(s).

5. Console (1) selon l'une des revendications précédentes, dans laquelle le système (15) de restitution audio présente au moins une unité de commande ou une zone (25) de rattachement d'une unité de commande, ladite unité de commande incluant, de préférence, un moyen numérique de mémorisation et/ou de reproduction comme, par exemple, un "iPod", un "iPhone" ou un appareil MP3.

6. Console (1) selon l'une des revendications précédentes, dans laquelle le bâti de support (2) est pourvu d'un cadre ou, respectivement, d'un corps central (3) respectivement destiné au montage ou à la suspension sur une monture au plafond, les multiples modules (4) étant respectivement installés ou fixés sur les côtés dudit cadre ou corps central (3).

7. Console (1) selon l'une des revendications précédentes, dans laquelle le bâti de support (2) revêt une forme allongée et est disposé pour l'essentiel verticalement, les multiples modules (4) étant placés en superposition.

8. Console (1) selon l'une des revendications précédentes, dans laquelle au moins l'un des modules présente un corps de base ou un boîtier (16) qui reçoit, et entoure au moins partiellement des parties du système (15) de restitution audio.

9. Console (1) selon l'une des revendications précédentes, dans laquelle une première colonne (10), disposée pour l'essentiel verticalement, se trouve sur un côté du bâti de support (2) et une seconde colonne (11), disposée pour l'essentiel verticalement et constituée de modules (4) respectivement placés en superposition, se trouve sur un côté opposé dudit bâti de support (2).

10. Système de support (30) conçu pour des appareils médicaux, ledit système de support étant respectivement destiné au montage ou à la suspension sur une monture au plafond (35) dans une salle de traitement, et comprenant une console (1) conforme à l'une des revendications précédentes.

11. Système de support (30) selon la revendication 10, équipé d'une monture (35) respectivement affectée au montage ou à la suspension dudit système à un plafond (D).

12. Système de support (30) selon la revendication 10 ou 11, dans lequel le système audio (15) est réalisé en vue de la sonorisation d'ambiance de la salle de traitement, et/ou dans lequel ledit système audio (15) est respectivement réalisé en vue de la restitution ou de la diffusion des sonorités respectivement bénéfiques, ou thérapeutiques, par l'intermédiaire de haut-parleurs intégrés dans des casques d'écoute, de façon telle que lesdites sonorités respectivement bénéfiques, ou thérapeutiques, soient accessibles uniquement à des auditeurs déterminés dans la salle de traitement.
